# FASCICULE DE BREVET EUROPEEN

(11) **EP 0 738 147 B1**
(45) Date de publication et mention de la délivrance du brevet: **05.04.2000**
(21) Numéro de dépôt: 95906378.5
(22) Date de dépôt: 09.01.1995
(51) Int. Cl.: A61K 31/425

(54) **APPLICATION DU RILUZOLE DANS LE TRAITEMENT DES MALADIES MITOCHONDRIALES**
VERWENDUNG VON RILUZOL ZUR BEHANDLUNG MITOCHONDRIALER ERKRANKUNGEN
APPLICATION OF RILUZOLE IN THE TREATMENT OF MITOCHONDRIAL DISEASES

(30) Priorité: 12.01.1994 FR 9400249
(43) Date de publication de la demande: 23.10.1996
(73) Titulaire: RHONE-POULENC RORER S.A., 92160 Antony (FR)
(72) Inventeur: DELUMEAU, Jean-Christophe, F-92160 antony (FR); MARTINET, Michel, F-75012 Paris (FR); REIBAUD, Michel, F-94000 Créteil (FR); STUTZMANN, Jean-Marie, F-94440 Villecresnes (FR)
(74) Mandataire: Savina, Jacques
(86) Numéro de dépôt international: FR9500023
(87) Numéro de publication internationale: WO9519170

(56) Documents cités:
- EP-A- 0 282 971
- EP-A- 0 374 041
- EP-A- 0 558 861
- WO-A-94/05275
- WO-A-94/06428
- EUR. NEUROPSYCHOPHARMACOL. (NETHERLANDS), 1993, VOL. 3, NO. 3, PAGE(S) 184-185, Meldrum B.S. 'Anti-excitatory amino acid approach in the treatment of neurodegenerative disorders'
- EUR. J. PHARMACOL., vol.250, no.3, 1993 pages 473 - 476 D. MARTIN ET AL. 'The neuroprotective agent riluzole inhibits release of glutamate and aspartate from slices of hippocampal area CA1'
- CURRENT OPINION IN NEUROLOGY & NEUROSURGERY, vol.5, no.5, 1992 pages 622 - 632 I. NONAKA 'Mitochondrial diseases' cité dans la demande
- NEUROLOGY, vol.43, no.12, 1993 pages 2484 - 2490 P.M. MATTHEWS 'Proton MR spectroscopic characterization of differences in regional brain metabolic abnormalities in mitochondrial encephalomyopathies'

## Description

La présente invention concerne une nouvelle application thérapeutique du riluzole ou les sels pharmaceutiquement acceptables de ce composé.

Le riluzole ou amino-2 trifluorométhoxy-6 benzothiazole est utile comme médicament anticonvulsivant, anxiolytique et hypnotique (brevet EP 50551), dans le traitement de la schizophrénie (EP 305276), dans le traitement des troubles du sommeil et de la dépression (EP 305277), dans le traitement des désordres cérébrovasculaires et comme anesthésique (EP 282971).

Il a maintenant été trouvé de manière surprenante que ce composé peut aussi être utilisé dans le traitement des maladies mitochondriales.

Les maladies mitochondriales sont des maladies dégénératives liées à différents mécanismes comme par exemple des anomalies de l'ADN mitochondrial (délétions, mutations ponctuelles, déplétions, duplications), des anomalies de l'ADN cellulaire codant pour des enzymes mitichondriales ou des éléments complexes macromoléculaires mitochondriaux, des causes acquises toxiques (MPTP, CO par exemple) ou médicamenteuses (chloramphénicol, AZT, acide acétylsalicylique par exemple). Parmi les maladies mitochondriales, on peut citer le syndrome de KEARNS-SAYRE, le syndrome MERRF (Myoclonic Epilepsy with Ragged Red Fibers), le syndrome MELAS (Mitochondrial Encephalopathy, Myopathy, Lactic Acidosis and Stroke-like episodes) et la maladie de LEBER (I. NONAKA, Current Opinion in Neurology and Neurosurgery, 5, 622 (1992)).

L'action du riluzole dans le traitement des maladies mitochondriales est démontrée par son effet protecteur dans le test au cyanure. Il est en effet connu que le cyanure de potassium exerce un effet toxique par inhibition de la cytochrome oxidase aa3, l'enzyme terminale de la chaîne de transport d'électrons au niveau mitochondrial. Ainsi, chez la souris, le cyanure de potassium (3 mg/kg IV) induit des contractions abdominales et des attaques toniques suivies de la mort dans les 20 secondes.

Des souris mâles adultes de 22-25 g (CD1 COBS, Charles River) stabulées à température et éclairement contrôlés reçoivent nourriture et eau de boisson ad libitum. A des groupes de 6 animaux on donne le véhicule ou le produit à étudier soit par voie intrapéritonéale 30 minutes avant l'administration intraveineuse bolus d'une dose léthale (3 mg/kg) de cyanure de potassium, soit par voie orale 1 heure avant l'administration du cyanure de potassium. Dans le groupe contrôle, toutes les souris meurent en 20 secondes alors que la DE50 du riluzole par voie IP est de 4,5 mg/kg et la DE50 du riluzole par voie per os est de 7,8 mg/kg.

L'action du riluzole dans le traitement des maladies mitochondriales est également démontrée par son effet sur le métabolisme mitochondrial du foie de rat.

L'activité métabolique de la mitochondrie est suivie par la consommation d'oxygène par polarographie à l'aide d'une électrode de Clark (commercialisée par BIOBLOCK). Le dispositif expérimental est constitué d'une chambre en verre, thermostatée à 27°C par un système d'eau chaude. Un barreau magnétique placé dans la chambre permet l'agitation en continu du milieu réactionnel et facilite l'établissement d'un équilibre entre l'oxygène dissous et le gaz diffusant au travers de la membrane de l'électrode. Les mitochondries, le substrat (succinate) et l'ADP (adénosine diphosphate) sont introduits dans la chambre réactionnelle par une petite ouverture située dans la partie supérieure de l'appareil. Une électrode de Clark est plongée dans le milieu réactionnel par un orifice hermétiquement fermé par un bouchon à vis. La consommation d'oxygène est tracée par un enregistreur connecté au moniteur type YSI model 5300 BIOLOGICAL OXYGEN MONITOR (commercialisé par BIOBLOCK).

Les mitochondries de foie de rat mâle SD sont isolées à +4°C par centrifugation différentielle selon la méthode décrite par APPELMANS et coll., Biochem. J., 59, 438-445 (1955). Le culot de mitochondries est repris par 1 à 3 ml de tampon saccharose 0,25 M et homogénéisé à la pipette.

La consommation d'oxygène par les mitochondries (1,2 mg de protéines par ml) est mesurée dans 5 ml de tampon de Chance composé de 12 mM de NaF, 26 mM de NaCl, 58 mM de KCl, 3 mM de substrat succinate pour se trouver dans l'état métabolique 4 de Chance-Williams (ou état 4 d'oxydo-réduction des mitochondries, CHANCE B., Energy liked functions of mitochondria, New York, Acad. Press 1963)). La température de 27°C correspond à une concentration en oxygène d'environ 250 µM. L'état métabolique 3 de Chance-Williams est déclenché par addition de 0,25 mM d'adénosine diphosphate dans des préparations témoins contenant 250 nM de riluzole.

La consommation moyenne d'oxygène par les mitochondries hépatiques témoins est de 1,63 µmoles/min/mg de protéines (3 essais). En présence de 250 nM de riluzole ajouté in vitro dans les mitochondries, la consommation d'oxygène est en moyenne égale à 2,12 µmoles/min/mg de protéines (3 essais). Ces résultats démontrent que le riluzole stimule la respiration mitochondriales d'environ 30 % par rapport aux contrôles.

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pour administration topique peuvent être par exemple des crèmes, lotions, collutoires, gouttes nasales ou aérosols.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :

| | |
|---|---|
| - Amino-2 trifluorométhoxy-6 benzothiazole | 50 mg |
| - Mannitol | 64 mg |
| - Cellulose microcristalline | 50 mg |
| - Polyvidone excipient | 12 mg |
| - Carboxyméthylamidon sodique | 16 mg |
| -Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) q.s.p. 1 comprimé pelliculé terminé à 245 mg | |

### Exemple B

On prépare, selon la technique habituelle, des gélules dosées à 50 mg de produit actif ayant la composition suivante :

| | |
|---|---|
| - Amino-2 trifluorométhoxy-6 benzothiazole | 50 mg |
| - Cellulose | 18 mg |
| - Lactose | 55 mg |
| - Silice colloïdale | 1 mg |
| - Carboxyméthylamidon sodique | 10 mg |
| -Talc | 10 mg |
| - Stéarate de magnésium | 1 mg |

### Exemple C

On prépare une solution injectable contenant 10 mg de produit actif ayant la composition suivante :

| | |
|---|---|
| - Amino-2 trifluorométhoxy-6 benzothiazole | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau | q.s.p. 4 cm3 |

Le procédé de préparation de médicaments utiles dans le traitement des maladies mitochondriales consiste à mélanger le riluzole ou les sels pharmaceutiquement acceptables de ce composé avec un ou plusieurs diluants et/ou adjuvants compatibles et pharmaceutiquement acceptables.

## Revendications

1. Application du riluzole ou les sels pharmaceutiquement acceptables de ce composé à la préparation de médicaments destinés au traitement des maladies mitochondriales.

2. Application selon la revendication 1 pour le traitement du syndrome de KEARNS-SAYRE, le syndrome MERRF, le syndrome MELAS et la maladie de LEBER.

3. Application selon l'une des revendications 1 et 2 pour obtenir un médicament comprenant 25 à 200 mg de riluzole.

## Claims

1. Use of riluzole or of the pharmaceutically acceptable salts of this compound in the preparation of medicinal products intended for the treatment of mitochondrial diseases.

2. Use according to claim 1, for the treatment of Kearns-Sayre syndrome, MERRF syndrome, MELAS syndrome or Leber's disease.

3. Use according to either of claims 1 and 2, to obtain a medicinal product comprising from 25 to 200 mg of riluzole.

## Patentansprüche

1. Verwendung von Riluzol oder der pharmazeutisch verträglichen Salzen dieser Verbindung zur Herstellung von Medikamenten zur Behandlung von Mitochondrialerkrankungen.

2. Verwendung nach Anspruch 1 zur Behandlung des KEARNS-SAYRE-Syndroms, des MERRF-Syndroms, des MELAS-Syndroms und der LEBER-Krankheit.

3. Verwendung nach einem der Ansprüche 1 und 2 zum Erhalt eines Medikaments mit 25 bis 200 mg Riluzol.
